# EUROPEAN PATENT SPECIFICATION

(11) **EP 3 137 099 B1**
(45) Date of publication and mention of the grant of the patent: **04.09.2019**
(21) Application number: 15718902.8
(22) Date of filing: 29.04.2015
(51) Int. Cl.: A61K 38/43, A61K 38/46, A61P 7/00

(54) **DNASE FOR USE IN TREATING VASO-OCCLUSIVE CRISIS**
DNASE ZUR VERWENDUNG IN DER BEHANDLUNG VON GEFÄSSOKKLUSIVER KRISE
DNASE POUR L'UTILISATION DANS LE TRAITEMENT D'UNE CRISE VASO-OCCLUSIVE

(30) Priority: 30.04.2014 EP 14305645; 11.07.2014 EP 14306133
(43) Date of publication of application: 08.03.2017
(73) Proprietor: INSERM (Institut National de la Santé et de la Recherche Médicale), 75013 Paris (FR); Université Paris Descartes, 75006 Paris (FR); Assistance Publique-Hôpitaux de Paris (APHP), 75004 Paris (FR)
(72) Inventor: BLANC-BRUDE, Olivier, F-75737 Paris cedex 15 (FR); LEJEUNE, Sylvain, F-75737 Paris cedex 15 (FR)
(74) Representative: Inserm Transfert
(86) International application number: PCT/EP2015/059306
(87) International publication number: WO 2015/165944

(56) References cited:
- WO-A1-97/25926
- WO-A1-2013/095215
- WO-A2-2012/166611
- CHEN G ET AL: "Heme-induced neutrophil extracellular traps (NETs) formation contributes to sickle cell disease pathogenesis", BLOOD, vol. 122, no. 21, 15 November 2013 (2013-11-15), page 184, XP009180715,
- MANNA S S ET AL: "Treatment of plastic bronchitis in acute chest syndrome of sickle cell disease with intratracheal rhDNase", ARCH DIS CHILD, vol. 88, 2003, pages 626-627, XP002731075,

## Description

### FIELD OF THE INVENTION:

The present invention relates to methods and pharmaceutical compositions for treating vaso-occlusive crisis.

### BACKGROUND OF THE INVENTION:

Sickle cell disease (SCD) is the first genetic disease and a public health challenge in France. SCD was designated public health priority by UNESCO in 2003, the French Ministry of Health in 2004 and WHO in 2006. Although a rare disease, the affliction is a worsening challenge for health and social services. A sharp increase in patient numbers put sickle cell disease at the top position of genetic diseases in French metropolitan areas, concerning 5,000 to 6,000 patients up to 18 yo, with an estimated 20,000 patients by 2020. Carrier frequency reaches 12% in French West Indies and rises in the USA and developed countries. Demographic trends and the systematic diagnosis at birth for ethnics at risk are partly responsible for this sharp increase in cases to treat. SCD, the most common genetic disease of our time, will soon become a public health challenge in western countries and France in particular.

SCD results from a point mutation (HbS) in the hemoglobin gene, causing hemoglobin to polymerize, mediating drastic and irreversible remodeling of red blood cells (RBC). Here, sickle cell disease refers to the homozygous HbSS phenotype.

In sickle cell disease and other hemolytic anemiae, RBC present many unique features including the expression of cell surface phosphatidylserine and adhesion receptors that are normally absent from the surface of mature healthy RBC. The remodeled RBC can aggregate and bind to each other, to other circulating cell types or to the vascular wall. Multicellular aggregates of excessive size fail to pass through small diameter vessels, forming vaso-occlusive plugs that stop the blood flow, particularly in capillaries and microvessels. Such vaso-occlusions and interuptions of perfusion cause episodes of ischemia in downstream tissues. Vaso-occlusions and ichemic events participate in one of the most deleterious manifestations of sickle cell disease, the vaso-occlusive crisis (VOC), which often requires hospitalization to help manage the extreme pain that it causes. But vaso-occlusions also participate in recurrent ischemic tissue injury on a chronic basis, resulting in progressive organ damage and eventually multiple organ failure.

The primary research focus in sickle cell disease has been the biology of the erythrocyte for the past decades, with the aim of blocking erythrocyte remodeling in order to prevent erythrocyte rigidification and aggregation, as well as VOC, intravascular hemolysis and anemia as a matter of consequence. However, the mechanisms that rule over the occurence of vaso-occlusions remain little understood. The physiopathology of SCD involves an intricate combination of circulating, inflammatory and cardiovascular factors that have only been envisaged recently.

The mechanisms of erythrocyte aggregation are little understood, even in healthy individuals. It is thought that erythrocytes aggregate in post-capillary venules, where a drop in shear stress occurs as the vessel diameter augments, facilitating contacts between erythrocytes. In health, discoid erythrocytes tend to come into contact on their flat sides and remain bound to form stacks. Il is believed that plasma fibrinogen participates in this type of post-capillary aggregation. However, in sickle cell disease, intravascular hemolysis and the break down of fragile erythrocytes liberates 'free' hemoglobin and heme into the bloodstream, and erythrocyte membrane fragments called microparticles (MP), which also carry erythrocyte cell surface receptors and include some of their contents.

There are virtually no specific drugs to treat or prevent VOC. Primary care for VOC is hospitalization to manage pain with powerful analgesics, combined with rehydration and oxygenation. No specific drug exists to prevent the occurrence or treat VOC. Long term hydroxy-urea treatment, since 1995, is sometimes prescribed to force the re-expression of fetal hemoglobin, in order to reduce sickle cell formation and ultimately reduce hemolysis. However, hydroxy-urea is only effective in about 40% of SCD patients, with presumptions of carcinogenesis and low fertility in the long term. The most severe cases of VOC are eligible for blood transfusion, with the associated exposure to viral infection and secondary hemochromatosis, and complications connected to transplantation. Hence, the burden of SCD on health institutions is bound to increase steeply worldwide. At this stage, gene medicine remains unable to correct the HbS mutations in adults. Advances in the prevention of RBC hemolysis have stagnated since the discovery of hydroxy-urea. There is thus a clear need for novel treatment strategies that will limit vaso-occlusions in order to abbreviate VOC, and also reduce the acute and chronic manifestations of the disease.

Plasma DNA isolation and characterization techniques have been discussed and refined [Jen, 2000 ; Stroun, 2001 ; Rykova, 2012]. In 2007, a study has reported the unexpected presence of an abnormal concentration of cell-free DNA in patient plasma [Vasavda, 2007]. In sickle cell disease patients at steady state, circulating cell-free DNA levels remained similar to those of resting healthy volunteers. However, circulating DNA augmented by up to 10 fold during CVO. Subsequently, another group validated the observation of increzased circulating DNA in the plasma of SCD patients, and particularly in CVO [Schimmel, 2013 Hematologica]. In addition, it was suggested that this DNA in SCD plasma may come primarily from inflammatory neutrophils releasing neutrophil extracellular traps (NETs). However, no specific physiopathological role of circulating DNA has yet been put forward in relation to RBC aggregation, RBC adhesion or vascular occlusions similar to those observed in chronic hemolytic anemiae, and in sickle cell disease in particular.

### SUMMARY OF THE INVENTION:

The present invention relates to methods and pharmaceutical compositions for treating vaso-occlusive crisis. In particular, the present invention is defined by the claims.

### DETAILED DESCRIPTION OF THE INVENTION:

The present invention is defined by the claims.

In SCD, there is an admitted connection between VOC and the emergence of circulating extracellular DNA. The physiopathological significance of this observation is entirely obscure. Here, the inventors have demonstrated *in vitro* that extracellular DNA causes RBC aggregation in blood from sickle cell disease patients, but not in healthy volunteers. The inventors have demonstrated that the blood of sickle cell disease patients presents signs of increased RBC agregation during VOC. The authors have demonstrated that addition of DNase to such aggregated blood rapidly dissociates aggregates and restore blood fluidity *in vitro.* The inventors have also demonstrated a practical method to administer DNase to obtain a rapid reperfusion of tissues subjected to vaso-occlusions in an experimental murine model of vaso-occlusions in mice with sickle cell disease. The invention thus represents a novel technique to treat patients with sickle cell disease, other major hemolytic syndromes, or diseases otherwise associated with RBC aggregation. The technique aims at dissolving RBC aggregates, allowing blood flow and tissue reperfusion, thereby limiting the duration of VOC and hospitalization as well as limiting the complications associated with vascular injury and ischemic episodes. The invention may also be useful as a prophylactic approach to help prevent the occurrence of RBC aggregation and vaso-occlusive crises. The invention may also be combined with other current therapeutic strategies for VOC, including blood transfusion programs and hydroxyurea.

Accordingly, the present invention also relates to a use of DNase as defined in the claims in a method of treating a vaso-occlusive crisis in a subject suffering from sickle cell disease comprising administering to the subject a therapeutically effective amount of a DNase.

As used herein the term sickle cell disease has its general meaning in the art and includes homozygous sickle cell disease (hemoglobin SS disease), doubly heterozygous sickle hemoglobin C disease (hemoglobin SC disease) and the sickle β-thalassemias.

The method of the present invention is particularly suitable for improving tissue reperfusion with blood, e.g. in Lung, kidney, heart, or brain tissue.

As used herein, the terms "treatment," "treating," and the like, refer to obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment," as used herein, covers any treatment of a disease in a mammal, particularly in a human, and can include: (a) preventing the disease or a symptom of a disease from occurring in a subject which may be predisposed to the disease but has not yet been diagnosed as having it (e.g., including diseases that may be associated with or caused by a primary disease); (b) inhibiting the disease or condition, i.e., arresting its development; and (c) relieving the disease, i.e., causing regression of the disease. In some embodiments, the treatment is a prophylactic treatment. The terms "prophylaxis" or "prophylactic use" and "prophylactic treatment" as used herein, refer to any medical or public health procedure whose purpose is to prevent a disease. As used herein, the terms "prevent", "prevention" and "preventing" refer to the reduction in the risk of acquiring or developing a given condition, or the reduction or inhibition of the recurrence or said condition in a subject who is not ill, but who has been or may be near a subject with the disease.

The term "DNase" has its general meaning in the art and refers to and includes all enzymes having a phosphodiesterase activity and the ability to hydrolyse DNA.

Any suitable DNase may be used in the present invention. The DNase will most preferably be a DNase I (EC 3.1.21.1). It may, however, in some embodiments be a DNase II (EC 3.1.22. 1). DNases occur in a number of species and any DNase capable of cleaving DNA may be used in the invention. The DNase may be from an animal source such as of bovine or porcine origin. It may be of plant, fungal, or microbial origin. However, typically and most preferably the DNase is of human origin and is preferably a recombinant human DNase. Commercially available DNase preparations such as Dornase™ and Pulmozyme™ may be used in embodiments of the invention.

In some embodiments, the DNase has DNA hydrolytic activity, for example in the case of DNase I it may hydrolyse DNA to give 5'-phosphate nucleotides and in the case of DNase II it may hydrolyse DNA to give 3' phosphate nucleotides. A fluorescence-based assay using, for example, Hoechst Stain may be used such as that which was detected in Labarce & Paiden, 1980, Anal. Biochem., 102:344-352 to assay for DNA hydrolysis. Hydrolytic activity may be assessed in a variety of ways known in the art including analytical polyacrylamide and agarose gel electrophoresis, hyperchromicity assay (Kunitz, J. Gen. Physiol. 33:349-362 (1950); Kunitz, J. Gen. Physiol. 33:363-377 (1950)) or methyl green assay (Kurnick, Arch. Biochem. 29:41-53 (1950); Sinicropi, et al., Anal. Biochem. 222:351-358 (1994)). The breakdown of DNA molecules from high to lower molecular weight forms may be monitored preferably by techniques such as agarose gel electrophoresis. Control experiments in the absence of the enzyme and/or in the presence of a protein known to possess DNase activity may be performed.

DNases are known to be prone to deamidation. Asparagine residues and in particular the asparagine at amino acid positions 7 and 74 of the mature human DNase I are prone to deamidation. This process converts the asparagine residues in question to aspartic acid or iso-aspartate residues. Deamidation reduces the activity of the enzyme and this is particularly the case for deamidation at the asparagine at amino acid position 74 of mature human DNase I. Techniques are available for removing deamidated forms of the enzymes to leave the amidated forms and these may be employed to prepare DNases for use in the invention. These techniques may include tentacle cation exchange (TCX) or affinity purification using DNA to purify the amidated forms of the enzyme which are still capable of binding DNA. A DNase preparation for use in the invention may typically comprise from 85 to 100%, preferably from 90 to 100%), more preferably from 95 to 100% and even more preferably from 99 to 100%) amidated, or partially amidated, enzyme by weight. In particular these figures refer to the amount of wholly amidated enzyme i.e. with all of the residues which are naturally amidated being amidated. They will typically have more than 95%>, preferably more than 99%> and even more preferably more than 99.9% of the DNase in an amidated form. In particular, these values refer to values at the time of production or to their values from one month to a year, preferably from two to six months and more preferably from three to four months after production. They may refer to the value during any point of the shelf-life of the product.

Variants of naturally occurring or known DNases may be used in the invention. Thus the term DNase encompasses such variants. The term "variants" refers to polypeptides which have the same essential character or basic biological functionality as DNase. The essential character of a DNase is phosphodiesterase activity and the ability to hydrolyse DNA. Assays for measuring DNA cleavage are described herein and these may be used to determine whether a variant has hydrolytic activity.

The sequence of the DNase may be modified so that it has extended half-life. For example the sequence of the enzyme may be changed to remove recognition sequences for certain proteases and in particular those derived from inflammatory cells.

The DNase employed in the invention may also be chemically modified to alter its properties such as, for example, its biological half-life. To achieve this covalent modifications may be introduced by reacting targeted amino acid residues of the native or variant DNase with an organic derivatising agent that is capable of reacting with selected amino acid side-chains or N- or C-terminal residues. Suitable derivatising agents and methods are well known in the art. Residues which in particular may be derivatised include cysteinyl residues (most commonly by reaction with α-haloacetates), histidyl residues (by reaction with diethylpyrocarbonate at pH 5.5-7.0), lysinyl and amino terminal residues (by reaction with succinic or other carboxylic acid anhydrides), arginyl residues (by reaction with reagents such as phenylglyoxal, 2,3-butanedione, 1,2-cyclohexanedione, and ninhydrin). Carboxyl side groups (aspartyl or glutamyl) may be selectively modified by reaction with carbodiimides or may be converted to asparaginyl and glutaminyl residues by reaction with ammonium ions. The covalent attachment of agents such as polyethylene glycol (PEG) or human serum albumin to the DNases may reduce their immunogenicity and/or toxicity of the variant and/or prolong its half-life and hence may be used in the invention. In some embodiments of the invention the DNase may be directly conjugated to the glycosaminoglycan or joined through an intermediate molecule.

In some embodiments, the agent is a molecule that interferes with the ability of deoxyribonucleic acid molecule to bind to receptors at the red blood cell surfaces. These molecules may be molecules that bind and block the receptors or molecules that bind and block deoxyribonucleic acid molecules. Molecules that block the receptor may be of low molecular weight, for example deoxyribonucleotides dimmers, phosphodeoxyribosyl pyrophosphate (PdRPP), deoxyribonucleotides or metal ion or high molecular weight such as peptides, polypeptides, proteins, antibodies or glycoproteins. Molecules that bind and block deoxyribonucleic acid molecules may be peptides, polypeptides, proteins, DNA binding proteins, nuclear proteins (as histons, protamines) or antibodies.

By a "therapeutically effective amount" of the agent is meant a sufficient amount of the agent to treat vaso-occlusive crisis in the subject. It will be understood, however, that the total daily usage of the agent is decided by the attending physician within the scope of sound medical judgment. The specific therapeutically effective dose level for any particular subject will depend upon a variety of factors including the disorder being treated and the severity of the disorder; activity of the specific compound employed; the specific composition employed, the age, body weight, general health, sex and diet of the subject; the time of administration, route of administration, and rate of excretion of the specific compound employed; the duration of the treatment; drugs used in combination or coincidential with the specific agent; and like factors well known in the medical arts. For example, it is well within the skill of the art to start doses of the compound at levels lower than those required to achieve the desired therapeutic effect and to gradually increase the dosage until the desired effect is achieved. However, the daily dosage of the agent may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the agent for the symptomatic adjustment of the dosage to the subject to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

The agent may be combined with pharmaceutically acceptable excipients, and optionally sustained-release matrices, such as biodegradable polymers, to form therapeutic compositions.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol ; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The agent can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungic agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

The agent may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the compounds of the invention formulated for parenteral administration, such as intravenous or intramuscular injection, other pharmaceutically acceptable forms include, e.g. tablets or other solids for oral administration; liposomal formulations ; time release capsules ; and any other form currently used.

The invention will be further illustrated by the following figures and examples. However, these examples and figures should not be interpreted in any way as limiting the scope of the present invention.

### FIGURES:

- **Figure 1:**: **Erythrocytes adhesion to NETs.**
- **Figure 2:**: **DNA-mediated RBC aggregation.**
- **Figure 3:**: **DNase-1 on MP-induced vaso-occlusive events**

### EXAMPLE:

### Material & methods :

**Animals:** We used 10-14 week old males of C57bl6J background unless otherwise indicated. We bred male SAD transgenic mice obtained from Dr Beuzard (9) and carrying a human hemoglobin *β* chain transgene with 3 mutations (*β*S *β*⁶Val, *β*S-Antilles *β*²³Ile and D-Punjab *β*¹²¹Glu). Genotype was confirmed through electrophoretic characterization of blood hemoglobins at the hematology department of the Hôpital Européen George Pompidou in Paris. Wild type (wt) animals were control littermates from the above colonies, or procured through Charles River, France. All procedures for study animal care and euthanasia followed the European Community standards (authorization #00577). Protocols were validated by the local Inserm ethics committee.

**Vaso-occlusive crises in mice:** We characterized the induction of VOC in SAD transgenic mice (18, 19) according to our previously published method [Bonnin, 2008; Sabaa, 2008; Camus, 2012], in response to the intravenous administration of purified MP derived from RBC (50000 to 100000 MP/mouse) [Camus, 2012]. Briefly, mice were anesthetized with isoflurane and monitored to prevent any cardiorespiratory depression. Mice were shaved and placed in the decubitus position on a heating blanket (38°C). We used a Vivid 7 echograph (GE Medical Systems®, Horten, Norway) equipped with a 12-MHz linear transducer (12L). The ultrasound probe was placed on the left side of the abdomen for examination of renal arteries, or in left lateral decubitus for cardiac output acquisition with transducer on the chest. Data were transferred on-line to an EchoPAC ultrasound image analysis workstation (GE Medical Systems®). Two-Dimensional ultrasound imaging of the abdomen in left-sided longitudinal B-mode allowed kidney width and height measurements. Color-coded blood flow detection by Doppler enabled renal arteries to be localized. A pulsed Doppler spectrum was recorded and peak systolic, end-diastolic and time-average mean BFV were measured in the renal artery, with Doppler beam angle correction. To calculate cardiac output, spatial flow profiles were analyzed in parasternal long-axis B-mode images of the pulmonary artery and BFV were measured as above. The following formula was applied: CO = [(V^{mean}.60). (π . (Dpa/2)²)], where CO is the cardiac output in ml/minute, V^{mean} is the mean time-averaged BFV in cm/s and Dpa is the pulmonary artery diameter in cm. Kidney sizes and BFV were determined by the same investigator, as means of 5 to 8 measurements. Repeatability for cardiac output measurements was verified. We controlled and confirmed that kidney size was similar between SAD and wild type mice. After sacrifice, kidneys were dissected, dehydrated, mounted in paraffin, sectioned and stained by Masson trichrome. Vascular congestion was observed by phase-contrast microscopy, as large erythrocyte aggregates occluding kidney capillaries and larger vessels. In some experiments, MP from SAD mouse RBC (50000 to 500000 MP/mouse) were injected in order to induce renal vaso-occlusions as previously reported [Camus, 2012]. In some experiments, purified DNase-1 was injected in the retro-orbital sinus at the indicated time (5 to 20 minutes) after the administration of MP and after a drop in renal perfusion was monitored by echo-Doppler. In this model, spontaneous renal reperfusion is thought to occur within 45 to 120 minutes after MP administration. Here, we aimed to monitor accelerated reperfusion of the kidneys after DNase administration.

### Results:

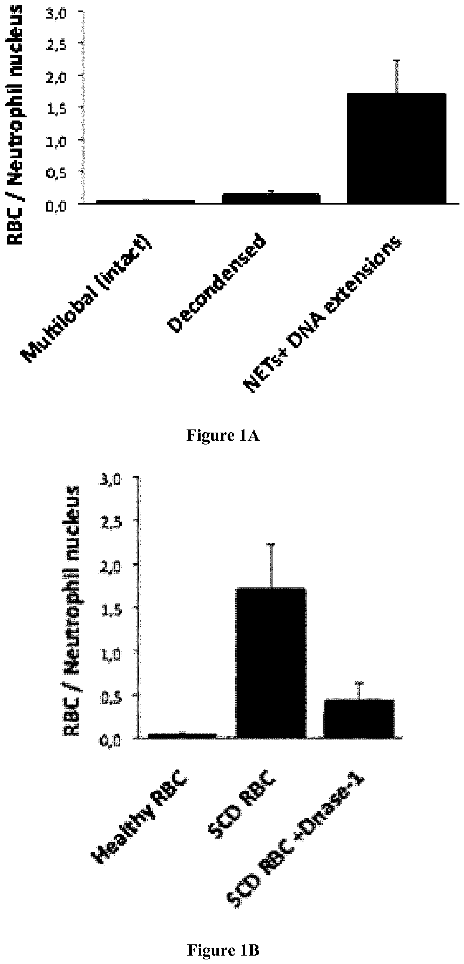

SCD RBC adhere preferentially to NETs (neutrophil extracellular traps, made of externalized DNA extensions), rather than neutrophils with intact nuclei, and little to neutrophils full of decondensed chromatin (Figure 1A). In contrast to SCD RBC, healthy RBC do not adhere to heme-activated neutrophils. Furthermore, DNase-1 treatment of heme-activated neutrophils degrades the externalized DNA, and triggers a concomittent reduction of SCD RBC adhesion (Figure 1B).

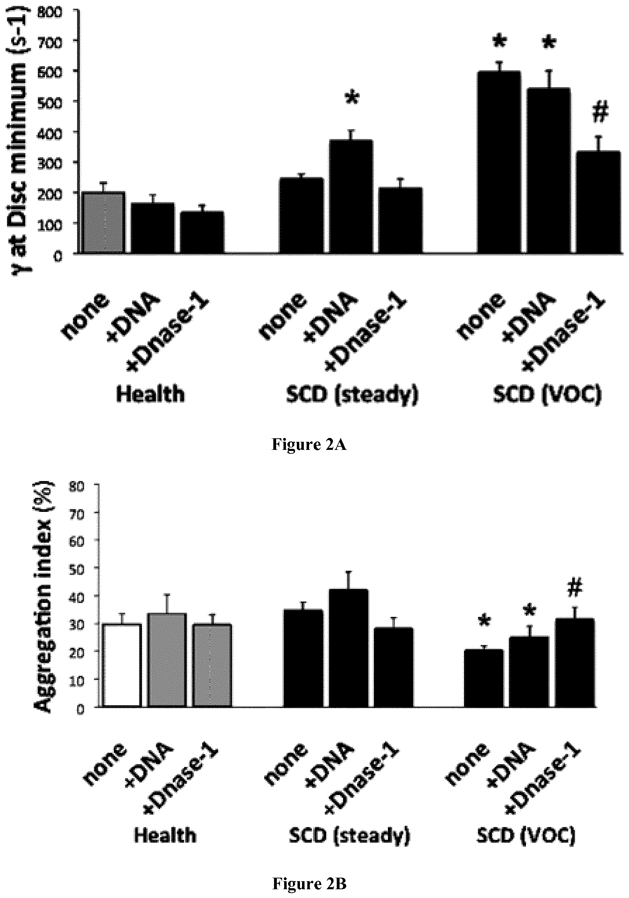

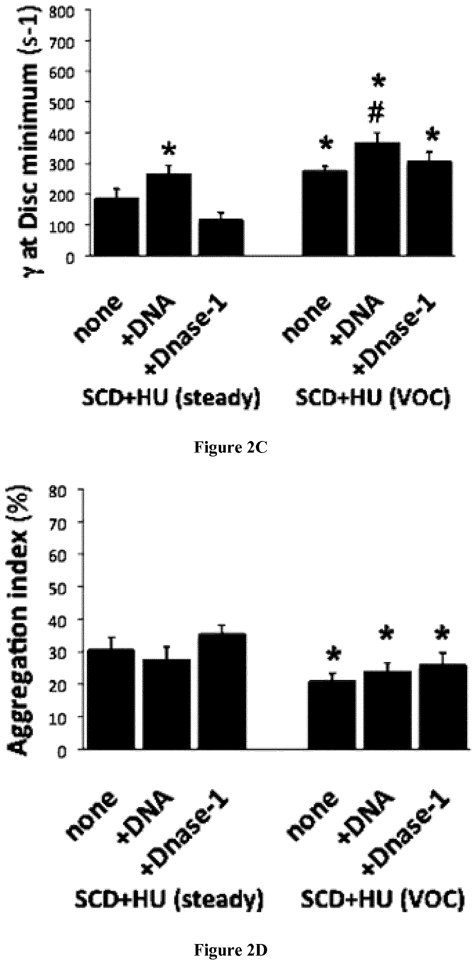

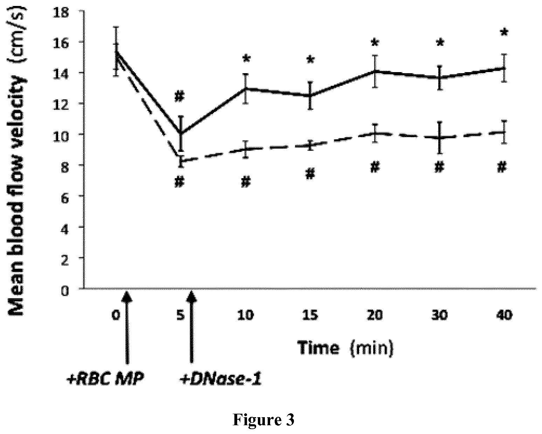

In other experiments, DNA is pro-aggregant in SCD and not in healthy individuals (Figure 2A and 2B). DNA is pro-aggregant in SCD at steady state, not during VOC (already maximal) (Figures 2A and 2B). This was particularly evident as an increase in the force that binds erythrocytes between each other (known as Gamma at Disc minimum in Figure 2A). DNase-1 is anti-aggregant in SCD (Figures 2A and 2B). DNA is pro-aggregant despite treatment of the patients with hydroxyurea (HU) (Figures 2C and 2D). DNase-1 is anti-aggregant in SCD patient blood during VOC, even under HU (Figures 2C and 2D). RBC microparticles (MP) induce vaso-occlusive crises within 5 to 15 minutes (Figure 3). Subsequently, DNase-1 administration accelerates kidney reperfusion, compared to controls (Figure 3).

### REFERENCES:

Throughout this application, various references describe the state of the art to which this invention pertains.
Bonnin, P., Sabaa, N., Flamant, M., Debbabi, H., and Tharaux, P.L. 2008. Ultrasound imaging of renal vaso-occlusive events in transgenic sickle mice exposed to hypoxic stress. Ultrasound Med Biol 34:1076-1084.
Camus S, Gausserès B, Bonnin P, Loufrani L, Grimaud L, Charue D, De Moraes JA, Renard JM, Tedgui A, Boulanger CM, Tharaux PL, Blanc-Brude OP. 2012. Erythrocyte microparticles can induce kidney vaso-occlusions in a murine model of sickle cell disease. Blood 120(25):5050-8.
Jen J1, Wu L, Sidransky D (2000). An overview on the isolation and analysis of circulating tumor DNA in plasma and serum. Ann N Y Acad Sci 906:8-12.
Kunitz (1950), Crystalline desoxyribonuclease; isolation and general properties; spectrophotometric method for the measurement of desoxyribonuclease activity. J. Gen. Physiol. 33:349-362.
Kunitz (1950), Crystalline desoxyribonuclease; digestion of thymus nucleic acid; the kinetics of the reaction. J. Gen. Physiol. 33:363-377.
Kurnick (1950). The determination of desoxyribonuclease activity by methyl green; application to serum. Arch. Biochem. 29:41-53.
Labarce & Paiden (1980). A simple, rapid, and sensitive DNA assay procedure. Anal. Biochem 102:344-352
Rykova EY, Morozkin ES, Ponomaryova AA, Loseva EM, Zaporozhchenko IA, Cherdyntseva NV, Vlassov VV, Laktionov PP (2012). Cell-free and cell-bound circulating nucleic acid complexes: mechanisms of generation, concentration and content. Expert Opin BiolTher 12 Suppl 1:S141-53.
Sabaa, N., de Franceschi, L., Bonnin, P., Castier, Y., Malpeli, G., Debbabi, H., Galaup, A., Maier-Redelsperger, M., Vandermeersch, S., Scarpa, A., et al. 2008. Endothelin receptor antagonism prevents hypoxia-induced mortality and morbidity in a mouse model of sickle-cell disease. J Clin Invest 118:1924-1933.
Schimmel M1, Nur E, Biemond BJ, van Mierlo GJ, Solati S, Brandjes DP, Otten HM, Schnog JJ, Zeerleder S; Curama Study Group (2013). Nucleosomes and neutrophil activation in sickle cell disease painful crisis. Haematologica 98(11):1797-803.
Sinicropi D1, Baker DL, Prince WS, Shiffer K, Shak S (1994). Colorimetric determination of DNase I activity with a DNA-methyl green substrate. Anal. Biochem. 222:351-358.
Stroun M1, Lyautey J, Lederrey C, Mulcahy HE, Anker P (2001). Alu repeat sequences are present in increased proportions compared to a unique gene in plasma/serum DNA: evidence for a preferential release from viable cells? Ann N Y Acad Sci 945:258-64.
Vasavda N, Ulug P, Kondaveeti S, Ramasamy K, Sugai T, Cheung G, Rees DC, Awogbade M, Bannister S, Cunningham J, Menzel S, Thein SL (2007). Circulating DNA: a potential marker of sickle cell crisis. Br J Haematol 139(2):331-6.

## Claims

1. A DNase for use in a method for the curative treatment of a vaso-occlusive crisis in a subject suffering from sickle cell disease.

2. The DNase for use according to claim 1 wherein the subject suffers from homozygous sickle cell disease, doubly heterozygous sickle hemoglobin C disease or sickle β-thalassemia.

3. The DNase for use according to claim 1 for improving tissue reperfusion with blood in lung, kidney, heart, or brain tissue.

4. The DNase for use according to claim 4 wherein the DNase is a DNase-I (EC 3.1.21.1) or a DNase-II (EC 3.1.22.1).

5. The DNase for use according to claim 4 wherein the DNase is of human origin and is preferably a recombinant human DNase.

## Patentansprüche

1. DNase zur Verwendung in einem Verfahren für eine Heilbehandlung einer gefäßokklusiven Krise in einem Subjekt, das an Sichelzellkrankheit leidet.

2. DNase zur Verwendung nach Anspruch 1, wobei das Subjekt an homozygoter Sichelzellkrankheit, doppelt heterozygoter Hämoglobin-C- Sichelzellkrankheit oder β-Thalassämie- Sichelzellkrankheit leidet.

3. DNase zur Verwendung nach Anspruch 1 zum Verbessern von Gewebe-Reperfusion mit Blut in Lungen-, Nieren-, Herz- oder Hirngewebe.

4. DNase zur Verwendung nach Anspruch 4, wobei die DNase eine DNase-I (EC 3.1.21.1) oder eine DNase-II (EC 3.1.22.1) ist.

5. DNase zur Verwendung nach Anspruch 4, wobei die DNase von menschlichem Ursprung ist und vorzugsweise eine rekombinante menschliche DNase ist.

## Revendications

1. DNase pour son utilisation dans un procédé de traitement curatif d'une crise vaso-occlusive chez un sujet souffrant de drépanocytose.

2. DNase pour son utilisation selon la revendication 1 dans laquelle le sujet souffre de drépanocytose homozygote, de drépanocytose hémoglobinose C double hétérozygote ou de thalassémie β falciforme.

3. DNase pour son utilisation selon la revendication 1 pour améliorer la reperfusion tissulaire avec du sang dans un tissu pulmonaire, rénal, cardiaque, ou cérébral.

4. DNase pour son utilisation selon la revendication 4 dans laquelle la DNase est une DNase I (EC 3.1.21.1) ou une DNase II (EC 3.1.22.1).

5. DNase pour son utilisation selon la revendication 4 dans laquelle la DNase est d'origine humaine et est de préférence une DNase humaine recombinante.
